(19) Europäisches Patentamt
European Patent Office
Office européen des brevets

(11) Veröffentlichungsnummer: **0 339 444**
**A1**

(12) # EUROPÄISCHE PATENTANMELDUNG

(21) Anmeldenummer: **89106971.8**

(22) Anmeldetag: **19.04.89**

(51) Int. Cl.⁴: **C07H 17/04 , C07H 15/26 , A61K 31/71**

(30) Priorität: **27.04.88 DE 3814138**

(43) Veröffentlichungstag der Anmeldung:
**02.11.89 Patentblatt 89/44**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Kretzschmar, Gerhard, Dr.**
**Ulmenweg 10**
**D-6236 Eschborn(DE)**
Erfinder: **Hammann, Peter, Dr.**
**Mörikestrasse 6**
**D-6233 Kelkheim Taunus(DE)**
Erfinder: **Düwel, Dieter, Dr.**
**Frankfurter Strasse 39**
**D-6238 Hofheim am Taunus(DE)**
Erfinder: **Grabley, Susanne, Dr.**
**Hölderlinstrasse 7**
**D-6240 Königstein Taunus(DE)**
Erfinder: **Kluge, Heinz, Dr.**
**Am Alten Birnbaum 10a**
**D-6238 Hofheim am Taunus(DE)**
Erfinder: **Wink, Joachim, Dr.**
**Bieberer Strasse 133**
**D-6050 Offenbach(DE)**

(54) **Elaiophylinderivate, Verfahren zu ihrer Herstellung und Verwendung als Heilmittel.**

(57) Verbindungen

EP 0 339 444 A1

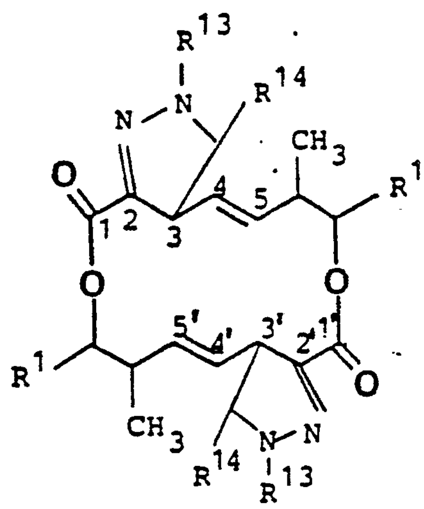

(I)

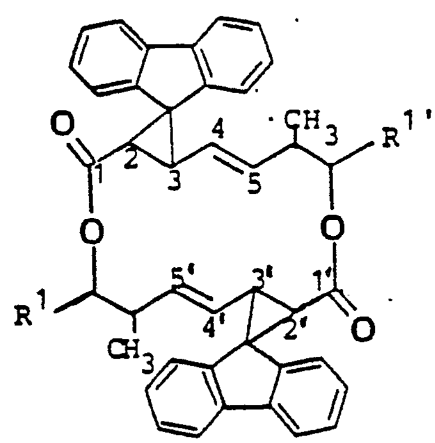

(II)

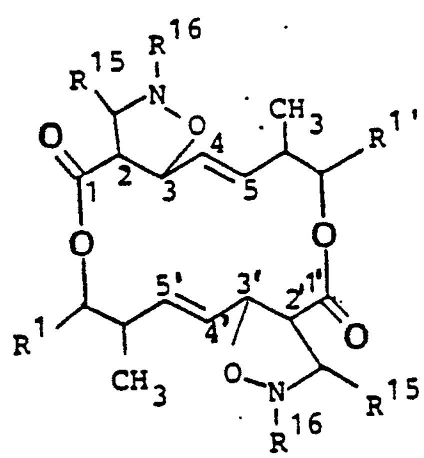

(III)

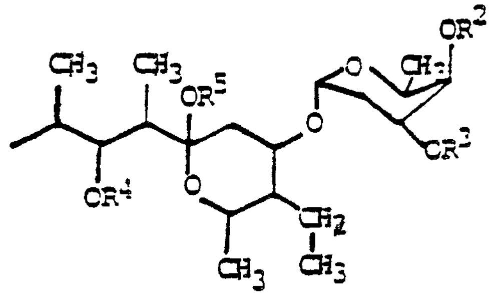

(IV)

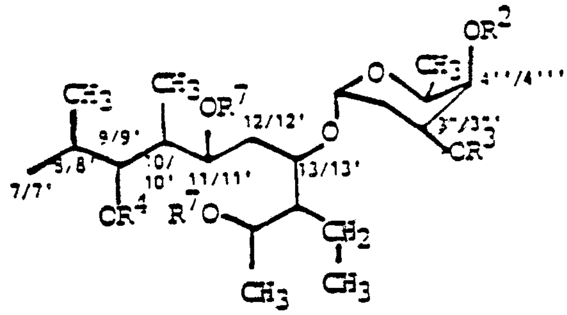

(V)

mit R¹ gleich IV und/oder V sind sehr gut wirksame Anthelmintika.
Sie werden erhalten durch Cycloaddition von Diazoverbindungen

$R^{14}CHN_2$ (XIII)

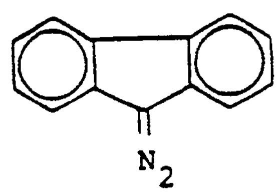

(XIV)

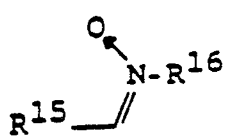

(XV)

an Elaiophylin XII

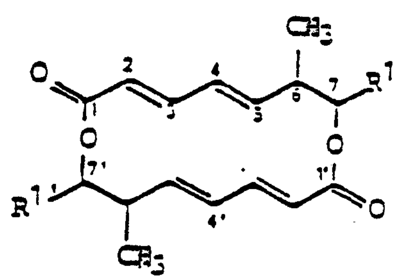

XII

## Elaiophylinderivate, Verfahren zu ihrer Herstellung und Verwendung als Heilmittel

Die Erfindung betrifft Elaiophylinderivate mit wesentlich verbesserter anthelminthischer Wirkung, Verfahren zu ihrer Herstellung und ihre Verwendung als Arzneimittel, insbesondere als Anthelminthika.

Elaiophylin (Formel XII; $R^1 = R^{1'} = IV$, $R^2R^3R^4R^5 = H$) wurde vor über 25 Jahren zuerst von Arcamone et al. (giorn. Microbiol. 7, 207, 1959) isoliert und später von Arai unter dem Namen Azalomycin B beschrieben. Kaiser et al. (Helv. Chim. Acta, 64 (1981), 407) isolierten Elaiophylin zusammen mit Nigericin und den Niphitricinen A und B aus Kulturen eines Stammes von Streptomyces violaceoniger.

Es wurde bereits vorgeschlagen (P 37 21 722.4 ≙ HOE 87/F 184), Elaiophylin als Anthelminthikum einzusetzen, da es insbesondere eine gute Wirkung gegen Magen-Darm-Strongyliden und Lungenwürmer entfaltet, von denen vor allem Haus- und Nutzti ere befallen werden.

Es wurde nun gefunden, daß durch chemische Derivatisierung des Elaiophylins die anthelminthische Wirkung noch gesteigert werden kann.

Einige Derivate des Elaiophylins sind bereits bekannt, jedoch liegt ihr pharmakologisches Wirkungsspektrum weitab von der hier beschriebenen anthelminthischen Wirkung; meist werden diese Derivate als Ulkustherapeutika eingesetzt.

Weitere Derivate des Elaiophylins sind vorgeschlagen in der deutschen Patentanmeldung P 37 36 960.1 (HOE 87/F 321).

Die Erfindung betrifft im speziellen Verbindungen der Formeln I, II und III

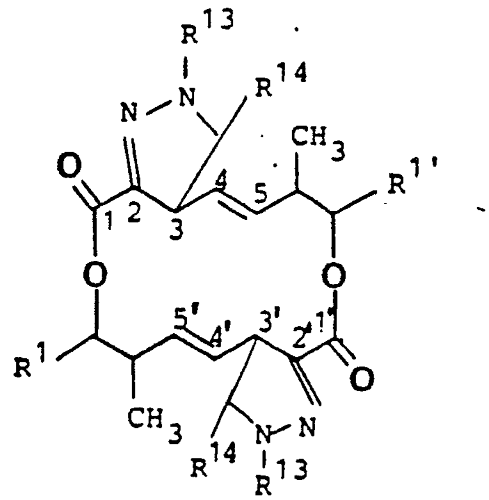

(I)

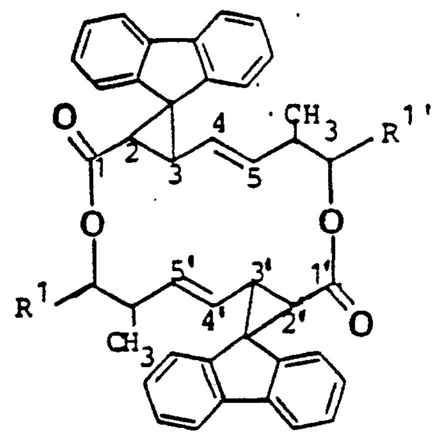

(II)

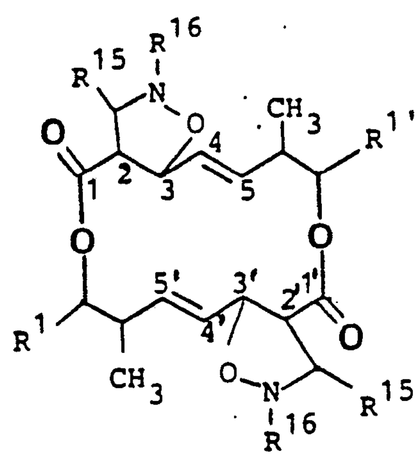

(III)

in welchen die folgenden Substituenten folgende Bedeutung haben:
$R^1$ ist ein Rest der Formel IV oder V,

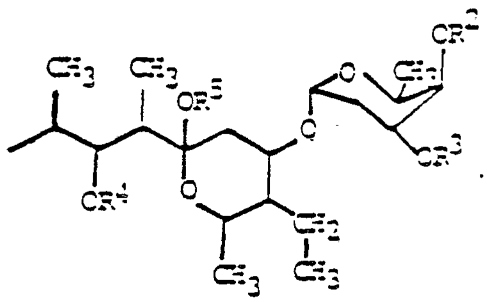

(IV)

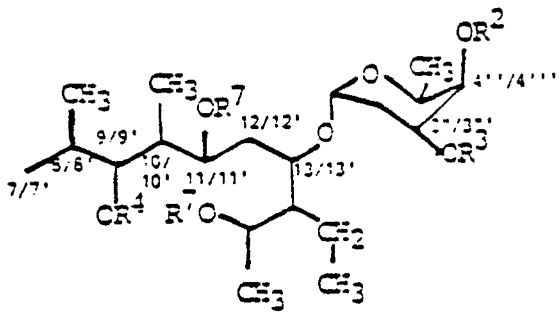

(V)

worin $R^2$ und $R^3$ gleich oder verschieden sind und Wasserstoff oder einen Rest der Formel VI oder VI′

$$-\overset{O}{\overset{\|}{C}}-R^6 \quad (VI) \qquad -\overset{O}{\overset{\|}{C}}-(CH_2)_nR^6 \quad (VI')$$

bedeuten, in der

n 1 bis 3 und

$R^6$ $C_1$-$C_{15}$-Alkyl, $C_2$-$C_{15}$-Alkenyl, $C_2$-$C_{15}$-Alkinyl, $C_3$-$C_9$-Cycloalkyl, Aryl- oder Heteroaryl bedeutet, wobei die Aryl- und Heteroarylreste gegebenenfalls halogen-, nitro-, cyano-, ($C_1$-$C_4$)acyloxy, $C_1$-$C_4$-alkyl- oder $C_1$-$C_4$-alkoxysubstituiert sind

und wobei für den Fall, daß $R^3$ Wasserstoff ist,

$R^2$ auch Wasserstoff ist,

oder worin

$R^2$ und $R^3$ gleich sind und

$C_1$-$C_4$-Alkyl, Benzyl, Allyl, MEM, MOM, SEM, Trimethylsilyl, Triethylsilyl, Dimethylphenylsilyl, Dimethylcyclohexylsilyl oder Dimethyltertiärbutylsilyl bedeutet

oder

$R^2$ und $R^3$ Sulfonsäureester der Formel $SO_2R^{10}$ bedeuten, in der

$R^{10}$ $C_1$-$C_{10}$-Alkyl, Phenyl oder p-Toluol bedeutet

oder

$R^2$ und $R^3$ einen Rest der Formel VII oder VII′ darstellen,

$$-\overset{X}{\overset{\|}{C}}-Z-R^{12} \quad (VII) \qquad -\overset{X}{\overset{\|}{C}}-Z-(CH_2)_n-R^{12} \quad (VII')$$

in der

n 1 bis 3

X Sauerstoff oder Schwefel

$R^{12}$ $C_1$-$C_{15}$-Alkyl, $C_3$-$C_9$-Cycloalkyl, Aryl, Pyridyl, Pyrimidyl oder Pyrazinyl bedeutet, wobei die Aryl-,

Pyridyl-, Pyrimidyl- oder Pyrazinylreste gegebenenfalls halogen-, nitro-, cyano- oder $C_1$-$C_4$-alkoxysubstituiert sind und

Z Sauerstoff, $>N-H$ oder einen Rest $>N-R^{12}$ oder $>N-(CH_2)_n-R^{12}$ bedeutet, wobei n und $R^{12}$ die oben angegebenen Bedeutungen haben

oder worin

$R^2$ und $R^3$ zusammen einen Rest der Formel VIII darstellen

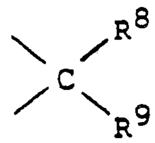

(VIII)

in der

$R^8$ und $R^9$ gleich oder verschieden sind und Wasserstoff oder unverzweigtes $C_1$-$C_{10}$-Alkyl bedeuten oder wobei

$R^8$ und $R^9$ eine Alkylenkette darstellt, die zusammen mit dem sie tragenden C-Atom einen 5-, 6- oder 7-gliedrigen Ring bildet

oder worin

$R^2$ und $R^3$ für den Fall, daß $R^1$ ein Rest der Formel IV ist, gleich sind und einen Rest der Formel IX darstellen,

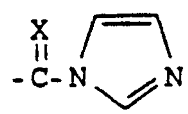

(IX)

in der

X Sauerstoff oder Schwefel bedeutet

oder

$R^2$ und $R^3$ für den Fall, daß $R^1$ ein Rest der Formel IV ist, zusammen einen Rest der Formel $C=X$ darstellen,

worin

X Sauerstoff oder Schwefel bedeutet

und worin

$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel VI oder VI′ ist,

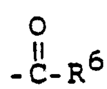 (VI) 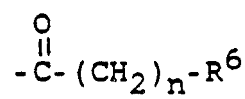 (VI′)

in der

n und $R^6$ die oben angegebenen Bedeutungen haben

und

$R^5$ Wasserstoff oder ein Rest der Formel $-(CH_2)_nR^6$ ist,

wobei

n und $R^6$ die oben angegebenen Bedeutungen haben

und worin

$R^7$ Wasserstoff oder - sofern $R^2$, $R^3$ und $R^4$ nicht gleichzeitig Wasserstoff bedeuten - ein Rest der Formel VI oder VI′ - wie oben definiert - ist,

und in denen

$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VIII bilden -verschieden substituiert sein können,

mit der Ausnahme, daß für $R^{1'}$ die Reste $R^2$ und $R^3$ im Rest der Formel IV oder V nicht gleichzeitig Wasserstoff sind, wenn $R^1$ ein Rest der Formel IV ist, in der $R^2$ und $R^3$ ein Rest der Formel IX ist oder in

der $R^2$ und $R^3$ zusammen einen Rest der Formel $> C=X$ bilden,
und in denen $R^{13}$ Wasserstoff oder einen Rest der Formel VI oder VI′ bedeutet, wobei für den Fall, daß mindestens einer der beiden Reste $R^2$ und $R^3$ Wasserstoff ist, der Rest $R^{13}$ gleichzeitig Wasserstoff sein muß,
und in denen
$R^{14}$ Wasserstoff oder einen Rest der Formel X oder XI bedeutet,

$$-\underset{\displaystyle R^{17}}{\underset{|}{CH}}-(CH_2)_nR^{17} \qquad (X) \qquad\qquad -CO_2(CH_2)_nR^{17} \qquad (XI)$$

worin $R^{17}$ Wasserstoff oder Methyl
und n null bis 3 bedeuten,
und in denen
$R^{15}$ unsubstituiertes Phenyl, Benzoyl, niederes Alkyl wie Ethyl und Propyl oder substituiertes Phenyl wie 4-Nitro-, 4-Chlor- und 4-Methoxyphenyl bedeutet,
und in denen
$R^{16}$ Methyl, unsubstituiertes Phenyl, Benzyl, Cyclohexyl oder substituiertes Phenyl wie 4-Chlor- und wie Methoxyphenyl bedeutet.

Bevorzugt sind Verbindungen I, II und III, in denen mindestens einer der Substituenten und Indices folgende Bedeutung hat:
$R^1$ und $R^{1'}$ wie in Anspruch 1 definiert, mit
$R^2$ und $R^3$ Wasserstoff oder einen Rest der Formel VI oder VI′ mit n = 1 bis 3 und $R^6$ gleich $(C_1-C_5)$-Alkyl, $(C_2-C_5)$-Alkenyl, $(C_2-C_6)$-Alkinyl, $(C_3-C_6)$-Cycloalkyl, Phenyl, Furyl, Thienyl, wobei Phenyl, Furyl und Thienyl unsubstituiert oder durch Halogen, $NO_2$, CN, $(C_1-C_4)$-Alkyl oder $(C_1-C_4)$-Alkoxy substituiert sind
und wobei für den Fall, daß $R^3$ Wasserstoff ist, $R^2$ auch Wasserstoff ist;
oder worin
$R^2$ und $R^3$ gleich sind und wie in Anspruch 1 definiert sind;
oder $R^2$ und $R^3$ Sulfonsäureester der Formel $SO_2R^{10}$ mit $R^{10}$ gleich $(C_1-C_5)$-Alkyl, Phenyl oder p-Toluyl bedeuten;
oder worin $R^2$ und $R^3$ einen Rest der Formel VII oder VII′ bedeuten mit n gleich 1 bis 3, X gleich Sauerstoff oder Schwefel, $R^{12}$ gleich $(C_1-C_5)$-Alkyl, $(C_3-C_6)$-Cycloalkyl, Phenyl, Pyridyl, Pyrimidyl oder Pyrazinyl, welche jeweils unsubstituiert oder nitro-, cyano- oder $(C_1-C_4)$-alkoxy substituiert sind; und mit Z gleich Sauerstoff, $\geq NH$ oder $\geq NR^{12}$ oder $\geq N-(CH_2)_n-R^{12}$
oder worin $R^2$ und $R^3$ zusammen einen Rest VIII darstellen mit $R^8$ und $R^9$ gleich Wasserstoff oder $(C_1-C_5)$-Alkyl,
oder worin $R^2$ und $R^3$ für den Fall, daß $R^1$ ein Rest der Formel IV ist, gleich sind und einen Rest der Formel IX darstellen wie in Anspruch 1 definiert,
oder worin $R^2$ und $R^3$ für den Fall, daß $R^1$ ein Rest der Formel IV ist, wie in Anspruch 1 definiert ist;
$R^4$, Sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest VI oder VI′ ist, wie in Anspruch 1 definiert;
$R^5$ Wasserstoff oder $-(CH_2)_nR^6$ mit n und $R^6$ wie in Anspruch 1 definiert,
$R^7$ Wasserstoff oder, sofern $R^2$, $R^3$ und $R^4$ nicht gleichzeitig Wasserstoff bedeuten, ein Rest der Formel VI oder VI′ wie in Anspruch 1 definiert,
$R^{13}$ Wasserstoff oder ein Rest der Formel VI oder VI′
$R^{14}$ wie in Anspruch 1 definiert
$R^{15}$ unsubstituiertes Phenyl oder Benzoyl oder $(C_1-C_5)$-Alkyl
$R^{16}$ wie in Anspruch 1 definiert.

Allgemein sind die Verbindungen der Formeln I, II und III durch Cycloaddition von Diazoalkanen und Nitronen an Elaiophylin und an Elaiophylinderivate erhältlich, in denen die 4 CC-Doppelbindungen des Makrodiolidrings vollständig intakt sind.

Die Diazoalkane und Nitrone reagieren regiospezifisch mit den in direkter Konjugation zur Lactoncarbonylgruppe ständigen $\alpha,\beta$-CC-Doppelbindungen des Elaiophylins und der Elaiophylinderivate unter Bildung der bezüglich des Makrodiolidrings symmetrischen Bisaddukte I, II und III. Dabei liefern Diazoalkane in Abhängigkeit von ihrer Struktur entweder die Bis-2-pyrazoline I oder die Biscyclopropane II. Mechanistisch gesehen resultieren die Produkte I aus den primär gebildeten 1-Pyrazolinen, die sich unter rascher 1,3-Wasserstoffverschiebung zu den 2-Pyrazolinen stabilisieren, während die Produkte II durch Stickstoffeliminierung der 1-Pyrazoline über einen Zwitterionen- oder Diradikalmechanismus entstehen.

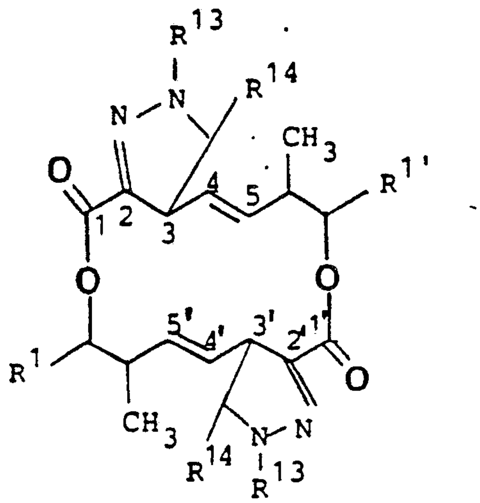

(I)

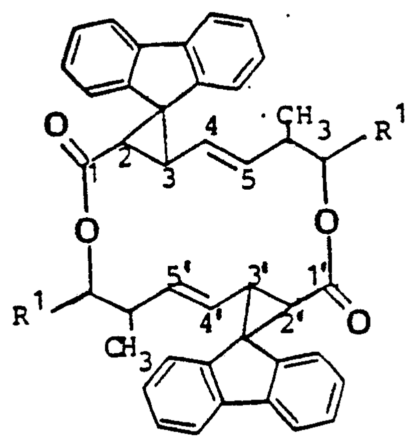

(II)

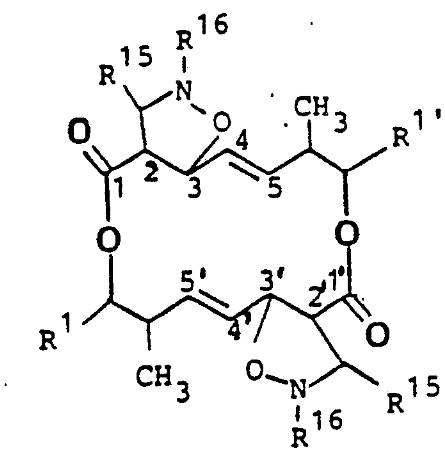

(III)

Die Cycloadditionsreaktionen sind ferner gekennzeichnet durch einen hoch stereoselektiven Aufbau der neuen Stereozentren in den 2,2′ und 3,3′-Positionen der Makrolide I, II und III: Die neuen Stereozentren des Makrodiolidrings werden innerhalb der NMR-Nachweisgrenze (ca. 5 %) konfigurativ eindeutig induziert.

Der hoch regio- und stereoselektive Verlauf dieser zweifachen 1,3-dipolaren Cycloadditionen von Diazoalkanen und Nitronen an Elaiophylin und 4-fach ungesättigte Elaiophylinderivate ist überraschend, weil

die in der Literatur beschriebenen 1,3-dipolaren Cycloaddition von Diazoalkanen an Alkene mit relativ geringer Stereoselektivität ablaufen. Die besten Resultate erreichten M. Oda et al. bei der Addition von Diazofluoren an 8-Phenylmenthylacrylate und -Fumarate mit Diastereoselektivitäten von 49/51 bis zu 95/5 und ee-Werten von 1 - 85 % (M. Oda, K. Okada, F. Samizo, Chemistry Letters 1987, 93.).

Die Stereoselektivität der Cycloaddition von Nitronen an Alkene wurde eingehend von Huisgen et al. untersucht und hängt wegen der Reversibilität dieser Reaktion stark von der Reaktionstemperatur ab (kinetische versus thermodynamische Reaktionskontrolle, vgl. R. Huisgen, H. Hauck, R. Grashey, H. Seidl, Chem. Ber. 102, 736 (1969)).

Besonders unerwartet ist auch der hoch regioselektive Verlauf dieser Cycloadditionen zugunsten der $\alpha,\beta$-CC-Doppelbindungen des Makrodiolidrings:
So findet der Primärangriff von Diazoalkanen an substituierten Butadienen, wie z. B. von Diazomethan an trans-2,4-Pentadiencarbonsäuremethylester, an der $\gamma,\delta$-CC-Doppelbindung des Dipolarophils statt, während die $\alpha,\beta$-CC-Doppelbindung erst bei der nachfolgenden Zweitaddition angegriffen wird (R. Huisgen, A. Ohta, J. Geittner, Chem. Pharm. Bull. 23 (11), 2735 (1975)).

Die durch Angriff der Diazoalkane oder Nitrone an nur einer der beiden $\alpha,\beta$-CC-Doppelbindungen des Makrodiolidrings gebildeten Zwischenprodukte sind im Zuge der Reaktion dünnschichtchromatographisch nachweisbar und können durch vorzeitiges Abbrechen der Reaktion abgefangen und isoliert werden. Dagegen sind die 1-Pyrazoline als reaktive Zwischenprodukte nicht faßbar.

Die Erfindung ist auch gerichtet auf ein Verfahren zur Herstellung von Elaiophylinderivaten der Formeln I, II und III.

Nach diesem Verfahren setzt man Elaiophylin (Formel XII, $R^1 = R^{1'} = IV$, $R^2$, $R^3$, $R^4$, $R^5 = H$)

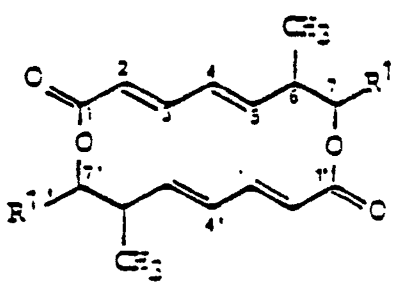

XII

oder eine Verbindung der Formel XII, in der $R^1$ und $R^{1'}$ gleich oder verschieden sind und einen Rest der Formel IV und/oder V darstellen, worin $R^2$, $R^3$, $R^4$, $R^5$ und $R^7$ die oben angegebenen Bedeutungen haben, um mit Verbindungen der Formeln XIII, XIV oder XV,

$R^{14}CHN_2$ (XIII)

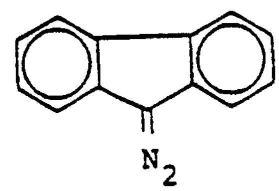

(XIV)

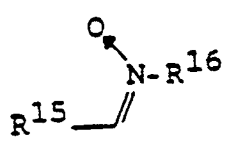

(XV)

wobei $R^{14}$, $R^{15}$ und $R^{16}$ die oben angegebenen Bedeutungen haben.

**Verfahren a)**

Mit Diazoalkanen der Formel XIII als Reagenzien erhält man die Produkte I, wobei $R^{13} = H$ bedeutet und $R^1$ und $R^{1'}$ Reste der Formeln IV bzw. V darstellen. Produkte I, bei denen $R^{13}$ Reste der Formeln VI oder VI' bedeuten, werden durch nachfolgende Acylierung mit einer Verbindung der Formel XVI oder XVI'

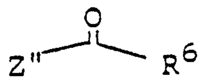 (XVI)

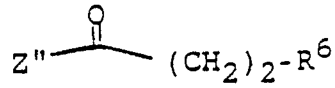 (XVI')

in der n und $R^6$ die oben zu Formel VI oder VI′ angegebenen Bedeutungen haben und Z″ Chlorid, Bromid, ein Imidazolid oder ein Säureanhydrid bedeutet, umsetzt.

**Verfahren b)**

Mit Diazofluoren (XIV) als Reagenz erhält man die Produkte II, wobei $R^1$ und $R^{1'}$ Reste der Formeln IV bzw. V darstellen.

**Verfahren c)**

Mit Nitronen der Formel XV als Reagenzien erhält man die Produkte der Formel III, wobei $R^1$ und $R^{1'}$ Reste der Formeln IV bzw. V darstellen und $R^{15}$ und $R^{16}$ die oben definierten Bedeutungen haben.

Unter Aryl werden aromatische Kohlenwasserstoffe, insbesondere Phenyl und Naphthyl verstanden und unter Heteroaryl, heteroaromatische Kohlenwasserstoffe, insbesondere Thiophen und Furan.

Unter Halogen werden Fluor, Chlor, Brom und Jod verstanden, unter MEM Methoxyethoxymethyl, unter MOM Methoxymethyl und unter SEM β-Trimethylsilylethoxymethyl. Die Substituenten $R^1$ und $R^{1'}$ können sowohl gleich als auch verschieden sein, d. h. es ist sowohl möglich, daß $R^1$ und $R^{1'}$ einen Rest der Formel IV oder V darstellen als auch, daß $R^1$ einen Rest der Formel IV darstellt und $R^{1'}$ einen Rest der Formel V darstellt (die Umkehr: $R^1$ = ein Rest der Formel V und $R^{1'}$ = ein Rest der Formel IV ist aufgrund der Symmetrie des Moleküls identisch mit $R^1$ = IV und $R^{1'}$ = V). Ebenso ist es - bis auf wenige Ausnahmen -möglich, Elaiophylinderviate mit in den Resten $R^1$ und $R^{1'}$ unterschiedlichen Resten $R^2$, $R^3$, $R^4$, $R^5$ bzw. $R^7$ einzusetzen. Dies ist unabhängig davon, ob die Reste $R^1$ und $R^{1'}$ beide einen Rest der Formel IV bzw. V darstellen, oder verschieden sind, also $R^1$ einen Rest der Formel IV und $R^{1'}$ einen Rest der Formel V darstellen.

Stellen $R^1$ und $R^{1'}$ jedoch einen Rest der Formel IV dar, so ist $R^5$ in beiden Substituenten $R^1$ und $R^{1'}$ gleich.

Der Substituent $R^4$ ist in den Resten $R^1$ und $R^{1'}$ immer identisch und kann nur dann von Wasserstoff verschieden sein, wenn auch die Substituenten $R^2$ und $R^3$ von Wasserstoff verschieden sind. Ebenso kann $R^2$ nur dann von Wasserstoff verschieden sein, wenn auch $R^3$ von Wasserstoff verschieden ist.

Im folgenden werden die Verfahren a) bis c), die es ermöglichen, die erfindungsgemäßen Elaiophylinderivate I, II und III herzustellen, näher beschrieben.

Bei der Verfahrensvariante a) geht man am besten so vor, daß man Elaiophylin (XII, $R^1$ = $R^{1'}$ = IV, $R^2$,$R^3$,$R^4$,$R^5$ = H) oder ein Elaiophylinderivat der Formel XII in einem geeigneten Lösungsmittel wie Methanol, Ethanol, Essigester, Dioxan, THF, Methylenchlorid, Diethylether, Dimethylformamid, Isopropanol, wäßrigem Alkohol, Benzol, Toluol, oder Mischungen der genannten Lösungsmittel in äquimolaren Mengen (pro reagierender CC-Doppelbindung) oder in bis zu einem 50-fachen Überschuß mit einer Verbindung der Formel XIII bis zur Beendigung der Reaktion umsetzt. Die Reaktionstemperaturen liegen zwischen -20° C und +140° C, vorzugsweise zwischen dem Festpunkt und dem siedepunkt des Lösungsmittels, insbesondere zwischen 0° C und 110° C. Die Reaktionszeiten betragen 0,5 bis 180 Stunden, bevorzugt 0,5 bis 48 Stunden, besonders bevorzugt 0,5 bis 8 Stunden. Die Beendigung der Reaktion kann beispielsweise mittels Dünnschichtchromatographie (DC-Kontrolle) bestimmt werden. Nach Abdestillieren des Lösungsmittels werden die Produkte durch Umkristallisieren oder Säulenchromatographie an Kieselgel gereinigt.

Die so gewonnenen Produkte können nun noch am Pyrazolinstickstoff acyliert werden (Einführung der Reste $R^{13}$), indem man sie mit einer Verbindung der Formel XVI oder XVI′ in äquimolaren Mengen oder in bis zu einem 50-fachen Überschuß, gegebenenfalls in einem inerten, aprotrischen Lösungsmittel wie Chloroform, Methylenchlorid, Tetrahydrofuran (THF), Essigester oder Dioxan bis zur Beendigung der Reaktion umsetzt, gegebenenfalls in Gegenwart einer Base, vorzugsweise Pyridin.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden (DC-Kontrolle).

Das als Ausgangssubstanz benötigte Elaiophylin kann beispielsweise nach dem in der deutschen Patentanmeldung P 37 21 722.4 (HOE 87/F 184) beschriebenen Verfahren hergestellt werden. Dabei fällt Elaiophylin als Fermentationsprodukt von Kulturen der Stämme Streptomyces violaceoniger DSM 4137 bzw. Streptomyces parvulus DSM 3816 an.

Die als Ausgangssubstanzen benötigten Elaiophylinderivate der Formel XII erhält man nach dem Verfahren der Deutschen Patentanmeldung P 37 36 960.1 (HOE 87/F 321).

Die Ausgangsverbindungen für die Verfahrensvariante a), die Verbindungen der Formel XVI und/oder XVI′ sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$.

Die Ausgangsverbindungen der Formel XIII sind, sofern nicht käuflich, nach zahlreichen literaturbekannten Methoden aus käuflichen Vorprodukten herstellbar.

Bei der Verfahrensvariante b) geht man am besten so vor, daß man Elaiophylin oder ein Elaiophylinderivat der Formel XII in äquimolaren Mengen (pro reagierender CC-Doppelbindung) oder in einem bis zu 50-fachen Überschuß, vorzugsweise mit 1 - 3 Äquivalenten 9-Diazofluoren XIV in einem geeigneten Lösungsmittel wie Methanol, Aceton, Essigester, THF, Dioxan, Ethanol, Isopropanol oder dergleichen umsetzt. Die Reaktionstemperaturen liegen dabei zwischen Zimmertemperatur und Siedepunkt des Lösungsmittels, insbesondere zwischen 20 und 100 °C. Die Reaktionszeiten betragen 0,5 bis 50 Stunden, bevorzugt 0,5 bis 5 Stunden. Die Beendigung der Reaktion kann beispielsweise mittels DC-Kontrolle bestimmt werden. Die Ausgangsverbindung XIV kann einfach durch Oxidation des käuflichen 9-Fluorenonhydrazons mit Quecksilberoxid oder nach anderen aus der Literatur bekannten Verfahren hergestellt werden.

Bei der Verfahrensvariante c) verfährt man am besten so, daß man Elaiophylin oder ein Elaiophylinderviat der Formel XII in äquimolaren Mengen (pro reagierender CC-Doppelbindung) oder in einem bis zu 50-fachen Überschuß, vorzugsweise mit 1 - 5 Äquivalenten eines Nitrons XV in einem geeigneten Lösungsmittel wie Methanol, Ethanol, Isopropanol, Dioxan Toluol, Xylol, Dimethylformamid, THF, Essigester oder dergleichen bis zur Beendigung der Reaktion umsetzt. Die Reaktionstemperaturen liegen zwischen Zimmertemperatur und Siedepunkt des Lösungsmittels, insbesondere zwischen 20 °C und 140 °C. Die Reaktionszeiten betragen 0,5 bis 50 Stunden, bevorzugt 0,5 bis 12 Stunden. Die Beendigung der Reaktion kann z. B. mittels DC-Kontrolle bestimmt werden.

Die Ausgangsverbindungen XV können nach Literaturvorschriften synthetisiert werden.

Die Herstellung der als Ausgangsverbindungen verwendeten Elaiophylinderivate XII und von Elaiophylin selbst (XII, $R^1$ = $R^{1'}$ = IV; $R^2$,$R^3$,$R^4$,$R^5$ = H) wird in den Patentanmeldungen P 37 36 960.1 (HOE 87/F 321) bzw. in P 37 21 722.4 beschrieben.

Dabei kommen die Verfahren 1 (Acylierung mit Carbonsäurederivaten zur Einführung von Acylresten $R^2$, $R^3$, $R^7$, $R^{13}$), Verfahren 2 (Acetalisierung der C-11-Position unter Einführung von $R^5$), Verfahren 3 (selektive Reduktion an C-11), Verfahren 4 (Einführung von Silylresten $R^2$ und $R^3$), Verfahren 5 (Acylierung mit Carbonsäurederivaten unter Katalyse mit 4-Dimethylaminopyridin) und Verfahren 6 (Acetalisierung der L-Desoxyfucosidreste in XII) zur Anwendung, die in Form allgemeiner Arbeitsvorschriften den Beispielen zur Darstellung der Cycloadditionsprodukte I, II und III nach den Verfahrensvorschriften a, b und c vorangestellt sind.

Als Ausgangsverbindungen können auch die von Kaiser et al. (Helv. Chim. Acta, 64 (1981), 407) beschriebenen reduzierten und teilreduzierten Halbacetale des Elaiophylins bei denen der 6-Ring am C11 bzw. am C11′ geöffnet ist, sowie das Tetraacetylderivat (3″,3‴,4″,4‴-Tetra-O-Acetyl) des Elaiophylins verwendet werden. Dies gilt auch für die von Yokura et al., in der Japanischen Offenlegungsschrift 61-36295 genannten 11,11′-O-Alkylderivate.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder (R)Sephadex LH 20 mit Lösungsmitteln wie niederen Alkanolen wie Methanol oder Chloroform oder Essigester oder Methanol/Chloroform-Mischungen, aber auch durch extraktive Methoden wie flüssig/flüssig-Extraktion oder fest/flüssig-Extraktion oder durch Kristallisation gereinigt werden.

Die Derivate des Elaiophylins zeigen anthelminthische Wirkung, insbesondere gegen Haemonchus, Trichostrongylus, Ostertagia, Cooperia, Chabertia, Strongyloides, Oesophagostomum, Hyostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden.

Die Elaiophylinderivate können grundsätzlich als solche in Substanz verabreicht werden. Bevorzugt ist die Verwendung in Mischung mit geeignetem Trägermaterial. Als Trägermaterial können die üblichen Futtermittelmischungen verwendet werden.

In den sich anschließenden Beispielen wird die Erfindung weiter erläutert. Prozentangaben beziehen sich auf das Gewicht und Mischungsverhältnisse bei Flüssigkeiten beziehen sich auf das Volumen, wenn keine anderen Angaben gemacht wurden.

## Beispiele

Allgemeine Verfahrensvorschriften für die Ausgangsverbindungen (XII)

### Verfahren 1

1 mmol Macrodiolid vom Elaiophylin-Typ (hierunter sind Verbindungen der Formel XII zu verstehen, in denen der Macrodiolidring nicht hydriert ist) werden in 10 ml Chloroform und 10 ml Pyridin gelöst und mit dem entsprechenden Carbonsäurederivat gerührt. Der Überschuß an Carbonsäurederivat wird mit 50 ml Wasser zerstört. Das Produkt wird durch dreimalige Extraktion mit Ethylacetat in die organische Phase überführt. Die organische Phase wird mit 0,1 N Salzsäure und Wasser gewaschen, über Natriumsulfat getrocknet und im Vakuum eingeengt. Durch Chromatographie an 100 g Kieselgel mit einem linearen Gradienten Ethylacetat : Hexan / 1 : 5 auf Ethylacetat (ca. 5 l) werden die Produkte gereinigt.

### Verfahren 2

1 mmol Macrodiolid vom Elaiophylin-Typ werden in 10 ml Alkohol gelöst und mit 70 mg $FeCl_3$ bei Zimmertemperatur gerührt. Nach der Zugabe von 100 ml Ethylacetat wird zweimal mit 50 ml einer gesättigten EDTA-Lösung (pH = 7, mit NaOH einstellen) und zweimal mit 50 ml Wasser gewaschen. Nach dem Trocknen der organischen Phase über Natriumsulfat wird im Vakuum bis einem festen Rückstand eingeengt.

### Verfahren 3

1 mmol Macrondiolid vom Elaiophylin-Typ wird in 100 ml Isopropanol mit 250 mg $NaBH_4$ für 24 Stunden bei Zimmertemperatur gerührt. Der Überschuß an Reduktionsmittel wird durch Zugabe von Aceton zerstört. Das Lösungsmittel wird im Vakuum abdestilliert und der Rückstand in Ethylacetat aufgenommen. Nach dem Ausschütteln mit Wasser wird die organische Phase über Natriumsulfat getrocknet und im Vakuum abdestilliert. Chromatographie an 100 g Kieselgel, mit einem linearen Gradienten von Ethylacetat : Hexan /1 : 5 auf Ethylacetat (bei Verbindungen mit $R^1 = R^{1'}$ und $R^2$, $R^3 \neq H$) bzw. Chloroform : Methanol / 40 : 1 auf Chloroform : Methanol / 1 : 1 (bei Verbindungen mit $R^1 = R^{1'}$ und $R^2$, $R^3$ = H) ergibt die reduzierten Verbindungen.

### Verfahren 4

1 mmol des Macrodiolids vom Elaiophylin-Typ wird in 20 ml Methylenchlorid suspendiert und unter Stickstoff mit 6 mmol SEM-Chlorid, MEM-Chlorid oder TBDMS-Chlorid und 2 ml (12 mmol) Diisopropylethylamin für 20 Stunden bei 25 °C gerührt. Nach der Zugabe von 5 ml Methanol wird das Lösungsmittel im Vakuum abdestilliert. Der Rückstand wird in Methylenchlorid aufgenommen und mit Wasser ausgeschüttelt. Nach dem Trocknen über Natriumsulfat wird das Lösungsmittel im Vakuum abgezogen. Chromatographie an Kieselgel (100 g) mit einem linearen Gradienten von Ethylacetat : Hexan / 1 : 5 auf Ethylacetat (ca. 5 l) ergibt die entsprechenden Verbindungen.

### Verfahren 5

1 mmol Macrodiolid vom Elaiophylin-Typ wird in 10 ml Dichlormethan und 10 ml Pyridin gelöst. Nach Zugabe eines Überschusses an Carbonsäureanhydrid und 1 mmol 4-Dimethylaminopyridin rührt man die

Mischung bei Zimmertemperatur, bis die Reaktion laut Dünnschichtchromatogramm beendet ist (ca. 2 bis 6 Stunden). Überschüssiges Anhydrid wird durch Zugabe von 5 ml Methanol unter Eiskühlung zerstört. Man verdünnt mit 50 ml Ethylacetat, extrahiert erst dreimal mit je 20 ml gesättigter Natriumhydrogencarbonatlösung, dann mit gerade soviel 0,1 N HCl-Lösung, daß die wäßrige Phase den pH-Wert 5 nicht überschreitet, und schließlich noch einmal mit 20 ml der gesättigten Natriumhydrogencarbonatlösung. Nach Trocknung der organischen Phase über Natriumsulfat und Einengen im Vakuum wird das Produkt entweder durch Kristallisation oder durch Säulenchromatographie an Kieselgel isoliert.

**Verfahren 6**

1 mmol Macrodiolid vom Elaiophylin-Typ wird mit einem Überschuß des betreffenden Aldehyds oder Ketons, die ihrerseits auch als Lösungsmittel verwendet werden können, bei Zimmertemperatur, gegebenenfalls unter Verdünnung mit einem Cosolvens wie Dichlormethan, Chloroform oder Tetrahydrofuran, in Gegenwart katalytischer Mengen einer wasserfreien Lewis-Säure wie $ZnCl_2$, $ZnI_2$, $FeCl_3$, $CuCl_2$, $CuSO_4$ gerührt, bis die Reaktion laut Dünnschichtchromatogramm beendet ist. Nach Verdünnen mit 50 ml Ethylacetat und dreimaligem Waschen mit je 20 ml einer gesättigten Natriumhydrogencarbonatlösung isoliert man das Produkt durch Säulenchromatographie an Kieselgel.

Die folgenden Beispiele dienen zur Illustration dieser allgemeinen Verfahrensvorschriften.

3″,3‴,4″,4‴-Tetra-O-(N-Phenyl)carbamoyl-elaiophylin

(Vorstufe für die Verbindung 22, Tabelle 3)

1,00 g (0,97 mmol) Elaiophylin und 2,40 g (20,1 mmol) Phenylisocyanat werden in 10 ml Pyridin und 20 ml Dichlormethan gelöst. Nach 6 h Rühren bei Zimmertemperatur wird das überschüssige Reagenz durch Rühren mit 100 ml einer gesättigten Natriumhydrogencarbonatlösung (ca. 30 min) zerstört. Die organische Phase wird getrocknet ($Na_2SO_4$), eingeengt und der Rückstand in Diethylether aufgeonommen. Man filtriert vom schwerlöslichen Carbanilid ab, engt die Etherlösung ein und chromatographiert den Rückstand an Kieselgel (ca. 100 g) mit 1 %igen Methanol in Dichlormethan. Aus Diethylether/n-Pentan kristallisieren 750 mg (51,4 %) des Produkts vom Schmelzpunkt 196 - 199 °C.
C (ber) 65,5 % / H (ber) 7,2 % / N (ber) 3,7 %
C (gef) 65,3 % / H (gef) 7,3 % / N (gef) 3,6 %
für $C_{82}H_{108}N_4O_{22}$ (1501,7)

11.11′-Di-O-Methylelaiophylin

(Vorstufe für Verbindungen 4 und 16 der Tabellen 1 und 3)

20,0 g (19,5 mmol) Elaiophylin werden in 100 ml wasserfreiem Methanol suspendiert und mit 600 mg wasserfreiem Eisen(III)chlorid versetzt.

Rührt man diese Mischung ca. 2 - 5 min bei 20 °C, so erhält man zunächst eine homogene orangegelbe Lösung. Nach Reiben mit einem Glasstab kristallisiert das Produkt spontan aus. Die Mutterlauge liefert nach Einengen und Kühlen noch weitere Produktfraktionen.
Ausbeute: 18,9 g (92 %) vom Schmelzpunkt 171 °C

| | |
|---|---|
| C (ber) 63,8 % | H (ber) 8,8 % |
| C (gef) 63,6 % | H (gef) 8,7 % |

3″,3‴,4″,4‴-Tetra-O-(tert.-Butyldimethylsilyl)elaiophylin

(Vorstufe für Verbindung 23 der Tabelle 3)

800 mg (0,78 mmol) Elaiophylin, 1,29 g (12 mmol) 2,6-Lutidin und 1,08 ml (4,7 mmol) Dimethyl-tert.-butylsilyl-triflat werden bei 0 °C in 10 ml wasserfreiem Dichlormethan gelöst. Nach 20 min entfernt man das Kältebad und läßt die Lösung innerhalb von 30 min auf Zimmertemperatur aufwärmen. Man verdünnt mit 200 ml Ether, wäscht dreimal mit je 50 ml einer gesättigten $NaHCO_3$-Lösung, trocknet über Natriumsulfat, engt ein und chromatographiert den öligen Rückstand an 100 g Kieselgel in Dichlormethan mit steigendem Gradienten an Diisopropylether.
Ausbeute: 474 mg (41 %) als fester Schaum mit
mit $[\alpha_D^{20}]$ -37° (c = 1, Methanol)

| | |
|---|---|
| C (ber) 63,2 % | H (ber) 9,8 % |
| C (gef) 63,0 % | H (gef) 10,1 % |

für $C_{78}H_{144}O_{18}Si_4$ (1482,3).

3,3‴,4″,4‴-Tetra-O-(2-Thienoyl)elaiphylin

(Vorstufe für die Verbindung 29 der Tabelle 5)

Eine Lösung von 0,60 g (0,58 mmol) Elaiophylin, 2,10 g (8,85 mmol) Thiophen-2-carbonsäure-anhydrid und 10 ml Pyridin in 20 ml Dichlormethan wird mit 500 mg (4,09 mmol) 4-Dimethylaminopyridin versetzt und 5 h bei 20 °C gerührt.

Überschüssiges Anhydrid zerstört man durch Zugabe von 5 ml wasserfreiem Methanol. Nach 10 min Rühren wird mit 100 ml Essigsäure-ethylester verdünnt, dreimal mit je 30 ml gesättigter $NaHCO_3$-Lösung gewaschen, getrocknet und eingeengt. Aus Methanol kristallisieren 0,56 g (65 %) des Produkts vom Schmelzpunkt 207 - 209 °C.

| | |
|---|---|
| C (ber) 60,6 % | H (ber) 6,6 % |
| C (gef) 60,6 % | H (gef) 6,4 % |

für $C_{74}H_{96}O_{22}S_4$ (1465,81)

**Allgemeine Verfahrensvorschriften für die Produkte (I), (II) und (III)**

**Verfahren a**

1 mmol Macrodiolid vom Elaiphylin-Typ (XII) werden in 50 ml Methanol und 1 ml Wasser gelöst und mit 200 ml einer etherischen Lösung versetzt, die ca. 5 - 15 Äquivalente des betreffenden Diazoalkans (XIII) enthält. Nach ca. 2-stündiger Reaktion bei Zimmertemperatur wird die Lösung eingeengt und der Rückstand umkristallisiert oder an Kieselgel chromatographiert.

Bei der Umsetzung mit den α-Diazoessigestern (XIII) verwendet man bis zu 15 Äquivalente Reagenz und erhitzt die Mischung ca. 25 h unter Rückfluß in 20 ml Tetrahydrofuran.

Die genauen Reaktionszeiten müssen im Einzelfall durch Verfolgung des Reaktionsverlaufs mittels Dünnschichtchromatographie an Kieselgel (Laufmittel z. B. Dichlormethan/Methanol-Mischungen) ermittelt werden.

**Verfahren b**

1 mmol Macrodiolid vom Elaiophylintyp (XII) und 6 mmol 9-Diazofluoren XIV werden 1,5 h in 15 ml Essigsäureethylester unter Rückfluß erhitzt. Nach Beendigung der Reaktion (DC-Kontrolle) engt man ein

und filtriert den Rückstand mit Dichlormethan über Kieselgel.

Aus dem rot gefärbten Eluat läßt sich die überschüssige Diazoverbindung zurückgewinnen. Das produkt erhält man durch Elution mit Essigsäure-ethylester in hoher Reinheit und Ausbeute.

## Verfahren c

1 mmol Macrodiolid vom Elaiophylin-Typ (XII) und 5 mmol des betreffenden Nitrons (XV) in 20 ml Toluol werden 2 h unter Rückfluß erhitzt. Nach Beendigung der Reaktion (DC-Kontrolle) wird eingeengt und das Produkt durch Säulenchromatographie an Kieselgel (Laufmittel: z. B. Dichlormethan/Methanol-Mischungen) vom überschüssigen Reagenz abgetrennt und gegebenenfalls durch Kristallisation weiter gereinigt.

In den Tabellen 1, 3 und 5 sind die gemäß den vorstehend beschriebenen Verfahren a, b bzw. c synthetisierten Verbindungen aufgeführt.

Die Substituenten im Rest $R^{1'}$ wurden mit $R^{2'}$, $R^{3'}$, $R^{4'}$, $R^{5'}$ und $R^{7'}$ bezeichnet. In diesen Tabellen sind die verschiedenen Substituenten der Elaiophylinderivate zwecks Identifizierung der Verbindungen sowie die jeweils eingesetzten Vorstufen, die nach einem der bezeichneten Verfahren 1 - 6 erhältlich sind, angegeben. In den Tabellen 2, 4 und 6 sind die jeweiligen Herstellungsverfahren, die individuellen Verfahrensparameter, die Reagenzien, die relative Konzentration des Reagenzes (bezogen auf die Ausgangsverbindung vom Elaiophylin-Typ), die Ausbeute sowie ausgewählte, charakteristische analytische Daten der erhaltenen Verbindungen angegeben. Eine Erklärung der in den Tabellen verwendeten Ausdrücke findet sich am Ende.

Tabelle 1

| (Produkte I/Verfahren a) | | | | | | | | | | | | | | | |
|---|---|---|---|---|---|---|---|---|---|---|---|---|---|---|---|
| Verb. | Vorstufe nach Verfahren | $R^1$ | $R^2$ | $R^3$ | $R^4$ | $R^5$ | $R^7$ | $R^{1'}$ | $R^{2'}$ | $R^{3'}$ | $R^{4'}$ | $R^{5'}$ | $R^{7'}$ | $R^{13}$ | $R^{14}$ |
| 1 | Ela | IV | H | H | H | H | - | IV | H | H | H | H | - | H | H |
| 2 | 1 | IV | Ac | Ac | H | H | - | IV | Ac | Ac | H | H | - | H | H |
| 3 | 1[tt] | IV | Ac | Ac | H | H | - | IV | Ac | Ac | H | H | - | Ac | H |
| 4 | 2 | IV | H | H | H | $CH_3$ | - | IV | H | H | H | $CH_3$ | - | H | H |
| 5 | Ela | IV | H | H | H | H | - | IV | H | H | H | H | - | H | $C_2H_5$ |
| 6 | Ela | IV | H | H | H | H | - | IV | H | H | H | H | - | H | $CO_2C_2H_5$ |
| 7 | 5 | IV | Bz | Bz | H | H | - | IV | Bz | Bz | H | H | - | H | H |
| 8 | 2+5 | IV | Bz | Bz | H | $CH_3$ | - | IV | Bz | Bz | H | $CH_3$ | - | H | H |
| 9[t] | 5 | IV | Ac | Ac | Ac | Ac | - | IV | Ac | Ac | Ac | Ac | - | H | H |
| 10 | 1 | IV | > C = O | | H | H | - | IV | > C = O | | H | H | - | H | H |
| 11 | 1 | IV | Val | Val | H | H | - | IV | Val | Val | H | H | - | H | H |
| 12 | 1 | IV | H | p-Br-Bz | H | H | - | IV | H | p-Br-Bz | H | H | - | H | H |
| 13 | 3 | V | H | H | H | H | H | IV | H | H | H | H | - | H | H |
| 14 | 3 | V | H | H | H | H | H | V | H | H | H | H | H | H | H |

Tabelle 2

(Produkte I/Verfahren a)

| Verbindung | Reaktionszeit (h) | Reaktionstemp. (°C) | Äquivalente XIII | Ausbeute (%) | Fp (°C) | [α ] (c=1,$CH_3OH$) | FAB-MS $MNa^+$ | C (ber) | C (gef) | H (ber) | H (gef) | N (ber) | N (gef) | Summenformel |
|---|---|---|---|---|---|---|---|---|---|---|---|---|---|---|
| 1 | 1,5 | 20 | 15 | 98 | 156-158 | -24,0 | | 60,6 | 61,0 | 8,4 | 8,5 | 5,0 | 5,2 | $C_{56}H_{92}N_4O_{18}$ |
| 2 | 2,5 | 20 | 15 | 78 | | -36,4 | | 59,8 | 59,5 | 7,8 | 7,5 | 4,3 | 4,1 | $C_{64}H_{100}N_4O_{22}$ |
| 3 | 24 | 20 | - | 77 | | +31,2 | | 59,9 | 59,8 | 7,7 | 7,8 | 4,1 | 3,9 | $C_{68}H_{104}N_4O_{24}$ |
| 4 | 2,5 | 20 | 15 | 89 | | +20,0 | | 61,2 | 60,9 | 8,5 | 8,8 | 4,9 | 4,7 | $C_{58}H_{96}N_4O_{18}$ |
| 5 | 1,0 | 20 | 7 | 62 | | | 1187 | 61,8 | 61,6 | 8,6 | 8,9 | 4,8 | 4,9 | $C_{60}H_{100}N_4O_{18}$ |
| 6 | 25 | 66 | 15 | 97 | | -104 | | 59,4 | 59,0 | 8,0 | 7,8 | 4,5 | 4,8 | $C_{62}H_{100}N_4O_{22}$ |
| 7 | 2,0 | 20 | 8 | 91 | | | 1547 | 66,1 | 59,8 | 7,1 | 7,4 | 3,6 | 3,4 | $C_{84}H_{108}N_4O_{22}$ |
| 8 | 2,0 | 20 | 10 | 79 | | | 1575 | 66,4 | 66,0 | 7,3 | 7,3 | 3,6 | 3,3 | $C_{86}H_{112}N_4O_{22}$ |
| 9 | 1,3 | 20 | 15 | 56 | | | 1467 | 59,8 | 59,5 | 7,5 | 7,6 | 3,8 | 3,6 | $C_{72}H_{108}N_4O_{26}$ |
| 10 | 1,5 | 20 | 15 | 83 | | | 1183 | 60,0 | 59,9 | 7,6 | 7,9 | 4,8 | 4,4 | $C_{58}H_{88}N_4O_{20}$ |
| 11 | 2,5 | 20 | 12 | 86 | | | 1467 | 63,1 | 63,4 | 8,6 | 9,0 | 3,8 | 3,5 | $C_{76}H_{124}N_4O_{22}$ |
| 12 | 1,5 | 20 | 15 | 74 | | | | 57,0 | 56,8 | 6,7 | 6,9 | 3,8 | 3,6 | $C_{70}H_{98}Br_2N_4O_{20}$ |
| 13 | 1,5 | 20 | 14 | 46 | | | 1133 | | | | | | | $C_{56}H_{94}N_4O_{18}$ |
| 14 | 1,5 | 20 | 12 | 63 | | | 1135 | | | | | | | $C_{56}H_{96}N_4O_{18}$ |

Tabelle 3

| (Produkte II/Verfahren b) | | | | | | | | | | | | | |
|---|---|---|---|---|---|---|---|---|---|---|---|---|---|
| Verb. | Vorstufe nach Verfahren | $R^1$ | $R^2$ | $R^3$ | $R^4$ | $R^5$ | $R^7$ | $R^1$ | $R^2$ | $R^3$ | $R^4$ | $R^5$ | $R^7$ |
| 15 | Ela | IV | H | H | H | H | - | IV | H | H | H | H | - |
| 16 | 2 | IV | H | H | H | $CH_3$ | - | IV | H | H | H | $CH_3$ | - |
| 17 | 1 | IV | Ac | Ac | H | H | - | IV | Ac | Ac | H | H | - |
| 18 | 1 + 2 | IV | Ac | Ac | H | $CH_3$ | - | IV | Ac | Ac | H | $CH_3$ | - |
| 19[1)] | 5 | IV | Ac | Ac | Ac | Ac | - | IV | Ac | Ac | Ac | Ac | - |
| 20 | 5 | IV | Bz | Bz | H | H | - | IV | Bz | Bz | H | H | - |
| 21 | 5 | IV | p-Br-Bz | p-Br-Bz | H | H | - | IV | p-Br-Bz | p-Br-Bz | H | H | - |
| 22 | 1 | IV | $C_7H_6NO$ | $C_7H_6NO$ | H | H | - | IV | $C_7H_6NO$ | $C_7H_6NO$ | H | H | - |
| 23 | 4 | IV | TBDMS | TBDMS | H | H | - | IV | TBDMS | TBDMS | H | H | - |
| 24 | 1 + 3 | V | Ac | Ac | H | - | H | V | Ac | Ac | H | - | H |

Tabelle 4

(Produkte II/Verfahren b)

| Verbindung | Reaktionszeit (h) | Reaktionstemp. (°C) | Äquivalente XIV | Ausbeute (%) | Fp (°C) | [α] (c = 1, $CH_3OH$) | FAB-MS $MNa^+$ | C (ber) | C (gef) | H (ber) | H (gef) | Summenformel |
|---|---|---|---|---|---|---|---|---|---|---|---|---|
| 15 | 2 | 70 | 3 | 99 | 194-196 | -27,0 | 1375 | 71,0 | 71,1 | 7,7 | 7,8 | $C_{80}H_{104}O_{18}$ |
| 16 | 1,5 | 77 | 3 | 92 | | | 1403 | 71,2 | 71,3 | 7,8 | 7,7 | $C_{82}H_{108}O_{18}$ |
| 17 | 1,5 | 77 | 3 | 86 | 179-181 | | | 69,4 | 69,1 | 7,4 | 7,0 | $C_{88}H_{112}O_{22}$ |
| 18 | 1,5 | 77 | 3 | 89 | | | 1571 | 69,7 | 69,4 | 7,5 | 7,7 | $C_{90}H_{116}O_{22}$ |
| 19 | 2 | 70 | 3 | 99 | | | | 68,2 | 68,2 | 7,1 | 6,8 | $C_{96}H_{120}O_{26}$ |
| 20 | 2 | 70 | 3 | 93 | | | 1791 | 73,3 | 73,4 | 6,8 | 6,8 | $C_{108}H_{120}O_{22}$ |
| 21 | 2 | 70 | 3 | 90 | | | | 62,2 | 61,9 | 5,6 | 5,7 | $C_{108}H_{116}Br_4O_{22}$ |
| 22 | 2 | 70 | 3 | 63 | | | | 70,8 | 70,7 | 6,8 | 7,0 | $C_{108}H_{124}N_4O_{22}$ |
| 23 | 2 | 70 | 3 | 74 | | | | 69,0 | 68,6 | 8,9 | 9,0 | $C_{104}H_{160}O_{18}Si_4$ |
| 24 | 2 | 70 | 3 | 69 | | | 1547 | | | | | $C_{88}H_{116}O_{22}$ |

Tabelle 5

(Produkte III/Verfahren c)

| Verb. | Vorstufe nach Verfahren | $R^1$ | $R^2$ | $R^3$ | $R^4$ | $R^5$ | $R^7$ | R' | R' | R' | R' | R' | R' | $R^{15}$ | $R^{16}$ |
|---|---|---|---|---|---|---|---|---|---|---|---|---|---|---|---|
| 25 | Ela | IV | H | H | H | H | - | IV | H | H | H | H | - | $C_6H_5$ | $C_6H_5$ |
| 26 | 1 | IV | Ac | Ac | H | H | - | IV | Ac | Ac | H | H | - | $C_6H_5$ | $C_6H_5$ |
| 27 | Ela | IV | H | H | H | H | - | IV | H | H | H | H | - | $C_6H_5$ | $CH_3$ |
| 28 | 1 | IV | Ac | Ac | H | H | - | IV | Ac | Ac | H | H | - | $C_6H_5$ | $CH_3$ |
| 29 | 5 | IV | 2-$C_5H_3OS$ | 2-$C_5H_3OS$ | H | H | - | IV | 2-$C_5H_3OS$ | 2-$C_5H_3OS$ | H | H | - | $C_6H_5$ | $C_6H_5$ |
| 30 | 5 | IV | Bz | Bz | H | H | - | IV | Bz | Bz | H | H | - | $C_6H_5$ | $CH_3$ |
| 31 | 2+5 | IV | Ac | Ac | H | $CH_3$ | - | IV | Ac | Ac | H | $CH_3$ | - | $C_6H_5$ | $C_6H_5$ |
| 32 | 2+5 | IV | Ac | Ac | H | $CH_3$ | - | IV | Ac | Ac | H | $CH_3$ | - | $C_6H_5$ | $CH_3$ |
| 33 | Ela | IV | H | H | H | H | - | IV | H | H | H | H | - | 4-Cl-$C_6H_4$ | $C_6H_5$ |
| 34 | 1 | IV | Ac | Ac | H | H | - | IV | Ac | Ac | H | H | - | 4-Cl-$C_6H_4$ | $C_6H_5$ |

Tabelle 6

(Produkte III/Verfahren c)

| Verbindung | Reaktionszeit (h) | Reaktionstemp. (°C) | Äquivalente XV | Ausbeut (%) | Fp (°C) | [α ] (c = 1, $CH_3OH$) | FAB-MS $MNa^+$ | C (ber) | C (gef) | H (ber) | H (gef) | N (ber) | N (gef) | Summenformel |
|---|---|---|---|---|---|---|---|---|---|---|---|---|---|---|
| 25 | 2 | 110 | 3 | 83 | | -91,0 | 1441 | 67,6 | 67,3 | 7,8 | 7,7 | 1,9 | 1,8 | $C_{80}H_{110}N_2O_{20}$ |
| 26 | 5 | 110 | 3 | 50 | 153 | -70,0 | 1609 | | | | | | | $C_{88}H_{118}N_2O_{24}$ |
| 27 | 3 | 110 | 4 | 67 | 148-150 | -61,8 | | | | | | | | $C_{70}H_{106}N_2O_{20}$ |
| 28 | 2,5 | 110 | 3 | 49 | 142-144 | -86,0 | 1485 | | | | | | | $C_{78}H_{114}N_2O_{24}$ |
| 29 | 5 | 110 | 2 | 52 | | | | 64,5 | 64,8 | 6,4 | 6,6 | 1,5 | 1,5 | $C_{100}H_{118}N_2O_{24}S_4$ |
| 30 | 2,5 | 110 | 3 | 56 | | | | 68,7 | 68,4 | 7,2 | 7,4 | 1,6 | 1,5 | $C_{98}H_{122}N_2O_{24}$ |
| 31 | 3 | 110 | 2 | 56 | | | 1637 | | | | | | | $C_{90}H_{122}N_2O_{24}$ |
| 32 | 2,5 | 110 | 3 | 47 | | | 1513 | | | | | | | $C_{80}H_{118}N_2O_{24}$ |
| 33 | 2 | 110 | 2,5 | 76 | | | | 64,5 | 64,8 | 7,3 | 7,0 | 1,8 | 1,6 | $C_{80}H_{108}Cl_2N_2O_{20}$ |
| 34 | 4 | 110 | 2,5 | 53 | | | | 64,8 | 63,6 | 7,3 | 7,0 | 1,8 | 1,5 | $C_{88}H_{116}Cl_2N_2O_{24}$ |

| Fußnoten zu den Tabellen 1 - 6 | |
|---|---|
| †) | Die $^{13}C$-NMR-Spektren in Pyridin-$d^5$ belegen, daß die peracylierten Verbindungen als geöffnete Hemiacetale in den 11,11'-Diketo-15,15'-Di-O-Acylformen vorliegen: $\delta$ ($C^{11},C^{11'}$) 210,1 pp . |
| ††) | Aus Verbindung Nr. 2 mit Verfahren 1 |
| †††) | Schmelzpunkte wurden nur von eindeutig kristallinen Substanzen bestimmt und sind nicht korrigiert. |

Die Verbindungen schmelzen in allgemeinen unter Zersetzung. Alle übrigen Substanzen sind amorphe Feststoffe. Die Strukturzuordnung basiert insbesondere auf $^1H$-, $^{13}C$-, 2D-NMR-, FAB-MS- und IR-Spektroskopie.

| Legende zu den Tabellen 1 - 6 | |
|---|---|
| Ela: | Elaiophylin $R^1,R^{1'}$ = IV und $R^2,R^3,R^4,R^5$ = H |
| Ac: | $-CO-CH_3$ |
| p-Br-Bz: | para-Brombenzoyl |
| Val: | $-CO-(CH_2)_3CH_3$ |
| TBDMS: | $(CH_3)_2SiC(CH_3)_3$ |
| $C_7H_6NO$: | $C_6H_5-NHC=O$ |
| Bz: | Benzoyl |
| 2-$C_5H_3OS$: | 2-Thienoyl |
| IV bzw. V: | in den Spalten unter $R^1$ und $R^{1'}$ ist angegeben, welchem Formeltyp (s. Formeln I, II und III) die Substituenten $R^1$ und $R^{1'}$ angehören. |

**Anthelminthische Wirkung der Elaiophylinderivate**

Die anthelminthische Wirkung der Elaiophylinderivate wurde an Lämmern mit 30 bis 40 kg Körpergewicht untersucht. Dazu wurden die Lämmer artifiziell mit Infektionsstadien von Labmagennematoden (Haemunchus contortus) infiziert. Nach Abschluß der Entwicklungszeit (Präpatenzperiode) der Nematoden erfolgte die Applikation der Elaiophylinderivate.

Durch koproskopische Untersuchungen vor und bis zu 14 Tagen nach der Applikation der Elaiophylinderivate und anschließender Sektion mit helminthologischer Aufarbeitung wurde die prozentuale Reduktion der Schaf-Nematoden ermittelt (s. Tabelle 7). Als Vergleichssubstanz diente Elaiophylin. Zusätzlich wurde auch noch die antibakterielle Aktivität der erfindungsgemäßen Elaiophylinderivate gegen Staph. aureus und Strept. pyogenes ermittelt. Überraschenderweise zeigen die erfindungsgemäßen Verbindung keine oder nur sehr geringe antibakterielle Aktivität. Gerade deshalb eignen sich die erfindungsgemäßen Verbindungen insbesondere für den Einsatz als Anthelminthika, da hier eine antibakterielle Wirkung unerwünscht ist.

Tabelle 7

| Verabr. Verb. aus Bsp. | Dosierung (mg/kg) | Reduktion v. H. contortus (%) | antibakterielle Aktivität (µg/ml) gegen | |
|---|---|---|---|---|
| | | | Stap. aureurs | Strept. pyogenes |
| Elaiophylin | 2,5 (s.c.) | 35-45 | 1,56 | 1,56 |
| | 5,0 (oral) | 70-95 | | |
| 1 | 2,5 (s.c.) | - | > 100 | > 100 |
| | 5,0 (oral) | 35-45 | | |
| 2 | 2,5 (s.c.) | - | > 100 | > 100 |
| | 5,0 (oral) | 40-50 | | |
| 4 | 2,5 (s.c.) | 30-40 | > 100 | > 100 |
| | 5,0 (oral) | 50-70 | | |
| 6 | 2,5 (s.c.) | 50-60 | > 100 | > 100 |
| | 5,0 (oral) | 50-60 | | |
| 3 | 2,5 (s.c.) | 30-40 | > 100 | 25,00 |
| | 5,0 (oral) | 70-80 | | |
| 15 | 2,5 (s.c.) | 20-30 | > 100 | > 100 |
| | 5,0 (oral) | 60-70 | | |
| 17 | 2,5 (s.c.) | 30-40 | > 100 | > 100 |
| | 5,0 (oral) | 50-70 | | |
| 19 | 2,5 (s.c.) | 20-30 | > 100 | > 100 |
| | 5,0 (oral) | 50-70 | | |
| 25 | 2,5 (s.c.) | 30-40 | > 100 | > 100 |
| | 5,0 (oral) | 30-40 | | |
| 26 | 2,5 (s.c.) | 20-30 | > 100 | > 100 |
| | 5,0 (oral) | 50-60 | | |
| 27 | 2,5 (s.c.) | 30-40 | 12,50 | 6,25 |
| | 5,0 (s.c.) | 70-80 | | |
| 28 | 2,5 (s.c.) | 30-40 | > 100 | > 100 |
| | 5,0 (oral) | 50-70 | | |

## Ansprüche

1. Verbindungen der Formeln I, II und III

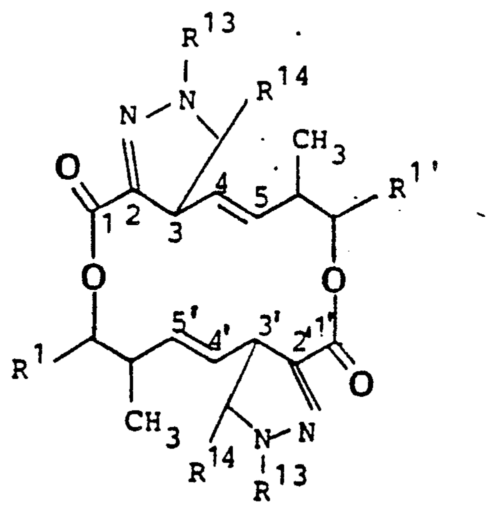

(I)

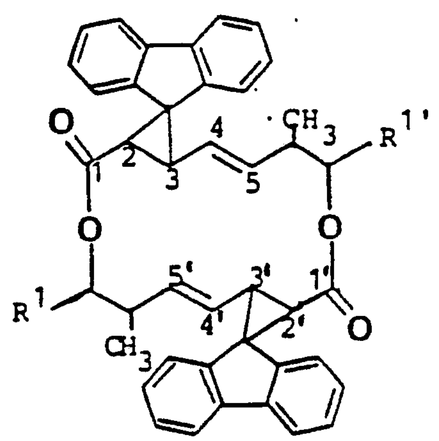

(II)

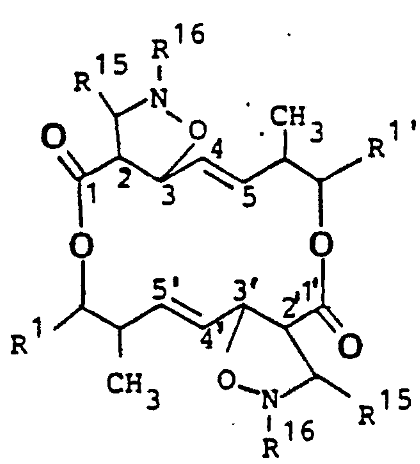

(III)

in denen die folgenden Substituenten und Indices folgende Bedeutung haben:
R' ist ein Rest der Formel IV oder V,

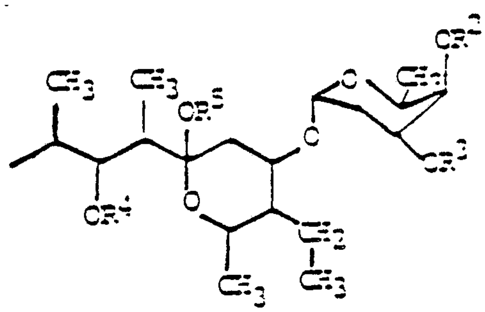

(IV)

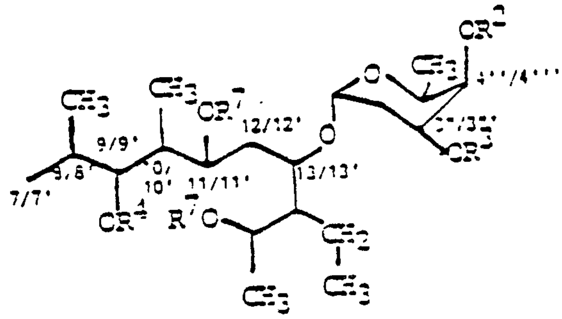

(V)

worin
$R^2$ und $R^3$ gleich oder verschieden sind und
Wasserstoff oder einen Rest der Formel VI oder VI′

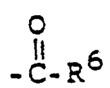 (VI) 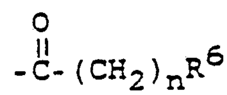 (VI')

bedeuten, in der
n 1 bis 3 und
$R^6$ $C_1$-$C_{15}$-Alkyl, $C_2$-$C_{15}$-Alkenyl, $C_2$-$C_{15}$-Alkinyl, $C_3$-$C_9$-Cycloalkyl, Aryl- oder Heteroaryl bedeutet, wobei die Aryl- und Heteroarylreste gegebenenfalls halogen-, nitro-, cyano-, ($C_1$-$C_4$)acyloxy, $C_1$-$C_4$-alkyl- oder $C_1$-$C_4$-alkoxysubstituiert sind
und wobei für den Fall, daß $R^3$ Wasserstoff ist, $R^2$ auch Wasserstoff ist,
oder worin
$R^2$ und $R^3$ gleich sind und
$C_1$-$C_4$-Alkyl, Benzyl, Allyl, MEM, MOM, SEM, Trimethylsilyl, Triethylsilyl, Dimethylphenylsilyl, Dimethylcyclohexylsilyl oder Dimethyltertiärbutylsilyl bedeutet
oder
$R^2$ und $R^3$ Sulfonsäureester der Formel $SO_2R^{10}$ bedeuten, in der
$R^{10}$ $C_1$-$C_{10}$-Alkyl, Phenyl oder p-Toluol bedeutet
oder
$R^2$ und $R^3$ einen Rest der Formel VII oder VII′ darstellen,

 (VII)  (VII')

in der
n 1 bis 3
X Sauerstoff oder Schwefel
$R^{12}$ $C_1$-$C_{15}$-Alkyl, $C_3$-$C_9$-Cycloalkyl, Aryl, Pyridyl, Pyrimidyl oder Pyrazinyl bedeutet, wobei die Aryl,

Pyridyl-, Pyrimidyl- oder Pyrazinylreste gegebenenfalls halogen-, nitro-, cyano- oder $C_1$-$C_4$-alkoxysubstituiert sind und

Z  Sauerstoff, >N-H oder einen Rest >N-$R^{12}$ oder >N- $(CH_2)_n$-$R^{12}$ bedeutet, wobei n und $R^{12}$ die oben angegebenen Bedeutungen haben

oder worin

$R^2$ und $R^3$ zusammen einen Rest der Formel VIII darstellen

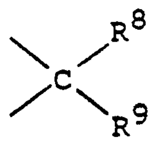

(VIII)

in der

$R^8$ und $R^9$ gleich oder verschieden sind und Wasserstoff oder unverzweigtes $C_1$-$C_{10}$-Alkyl bedeuten oder wobei

$R^8$ und $R^9$ eine Alkylenkette darstellt, die zusammen mit dem sie tragenden C-Atom einen 5-, 6- oder 7-gliedrigen Ring bildet

oder worin

$R^2$ und $R^3$ für den Fall, daß $R^1$ ein Rest der Formel IV ist, gleich sind und einen Rest der Formel IX darstellen,

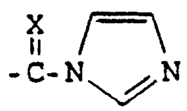

(IX)

in der

X Sauerstoff oder Schwefel bedeutet

oder

$R^2$ und $R^3$ für den Fall, daß $R^1$ ein Rest der Formel IV ist, zusammen einen Rest der Formel > C=X darstellen, worin

X Sauerstoff oder Schwefel bedeutet

und worin

$R^4$ sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest der Formel VI oder VI' ist,

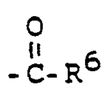 (VI) 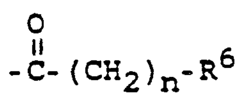 (VI')

in der

n und $R^6$ die oben angegebenen Bedeutungen haben

und

$R^5$ Wasserstoff oder ein Rest der Formel $-(CH_2)_nR^6$ ist,

wobei

n und $R^6$ die oben angegebenen Bedeutungen haben

und worin

$R^7$ Wasserstoff oder - sofern $R^2$, $R^3$ und $R^4$ nicht gleichzeitig Wasserstoff bedeuten - ein Rest der Formel VI oder VI' - wie oben definiert - ist,

und in denen

$R^{1'}$ die gleiche Bedeutung wie $R^1$ hat, wobei die beiden Reste $R^1$ und $R^{1'}$ sowohl gleich als auch - bis auf die Reste $R^4$ und $R^5$ und für den Fall, daß $R^2$ und $R^3$ zusammen einen Rest der Formel VIII bilden -verschieden substituiert sein können,

mit der Ausnahme, daß für $R^{1'}$ die Reste $R^2$ und $R^3$ im Rest der Formel IV oder V nicht gleichzeitig Wasserstoff sind, wenn $R^1$ ein Rest der Formel IV ist, in der $R^2$ und $R^3$ ein Rest der Formel IX ist oder in der $R^2$ und $R^3$ zusammen einen Rest der Formel > C=X bilden,

und in denen $R^{13}$ Wasserstoff oder einen Rest der Formel VI oder VI′ bedeutet, wobei für den Fall, daß mindestens einer der beiden Reste $R^2$ und $R^3$ Wasserstoff ist, der Rest $R^{13}$ gleichzeitig Wasserstoff sein muß,
und in denen
$R^{14}$ Wasserstoff oder einen Rest der Formel X oder XI bedeutet,

$$-\underset{\substack{|\\R^{17}}}{CH}-(CH_2)_nR^{17} \quad (X) \qquad -CO_2(CH_2)_nR^{17} \quad (XI)$$

worin $R^{17}$ Wasserstoff oder Methyl
und n null bis 3 bedeuten,
und in denen
$R^{15}$ unsubstituiertes Phenyl, Benzoyl, niederes Alkyl wie Ethyl und Propyl oder substituiertes Phenyl wie 4-Nitro-, 4-Chlor- und 4-Methoxyphenyl bedeutet,
und in denen
$R^{16}$ Methyl, unsubstituiertes Phenyl, Benzyl, Cyclohexyl oder substituiertes Phenyl wie 4-Chlor- und wie Methoxyphenyl bedeutet.

2. Verbindungen nach Anspruch 1, dadurch gekennzeichnet, daß mindestens einer der Substituenten oder Indices folgende Bedeutung hat:
$R^1$ und $R^{1'}$ wie in Anspruch 1 definiert, worin
$R^2$ und $R^3$ Wasserstoff oder einen Rest der Formel VI oder VI′ mit n = 1 bis 3 und $R^6$ gleich $(C_1-C_5)$-Alkyl, $(C_2-C_5)$-Alkenyl, $(C_2-C_6)$-Alkinyl, $(C_3-C_6)$-Cycloalkyl, Phenyl, Furyl, Thienyl, wobei Phenyl, Furyl und Thienyl unsubstituiert oder durch Halogen, $NO_2$, CN, $(C_1-C_4)$-Alkyl oder $(C_1-C_4)$-Alkoxy substituiert sind und wobei für den Fall, daß $R^3$ Wasserstoff ist, $R^2$ auch Wasserstoff ist;
oder worin
$R^2$ und $R^3$ gleich sind und wie in Anspruch 1 definiert sind;
oder $R^2$ und $R^3$ Sulfonsäureester der Formel $SO_2R^{10}$ mit $R^{10}$ gleich $(C_1-C_5)$-Alkyl, Phenyl oder p-Toluyl bedeuten;
oder worin $R^2$ und $R^3$ einen Rest der Formel VII oder VII′ bedeuten mit n gleich 1 bis 3, X gleich Sauerstoff oder Schwefel, $R^{12}$ gleich $(C_1-C_5)$-Alkyl, $(C_3-C_6)$-Cycloalkyl, Phenyl, Pyridyl, Pyrimidyl oder Pyrazinyl, welche jeweils unsubstituiert oder nitro-, cyano- oder $(C_1-C_4)$alkoxy substituiert sind; und mit Z gleich Sauerstoff, $\doteq$NH oder $\doteq NR^{12}$ oder $\doteq N-(CH_2)_n-R^{12}$
oder worin $R^2$ und $R^3$ zusammen einen Rest VIII darstellen mit $R^8$ und $R^9$ gleich Wasserstoff oder $(C_1-C_5)$-Alkyl,
oder worin $R^2$ und $R^3$ für den Fall, daß $R^1$ ein Rest der Formel IV ist, gleich sind und einen Rest der Formel IX darstellen wie in Anspruch 1 definiert,
oder worin $R^2$ und $R^3$ für den Fall, daß $R^1$ ein Rest der Formel IV ist, wie in Anspruch 1 definiert ist;
$R^4$, sofern $R^2$ und $R^3$ Wasserstoff sind, Wasserstoff bedeutet oder für den Fall, daß $R^2$ und $R^3$ nicht Wasserstoff bedeuten, Wasserstoff oder ein Rest VI oder VI′ ist, wie in Anspruch 1 definiert;
$R^5$ Wasserstoff oder $-(CH_2)_nR^6$ mit n und $R^6$ wie in Anspruch 1 definiert,
$R^7$ Wasserstoff oder, sofern $R^2$, $R^3$ und $R^4$ nicht gleichzeitig Wasserstoff bedeuten, ein Rest der Formel VI oder VI′ wie in Anspruch 1 definiert,
$R^{13}$ Wasserstoff oder ein Rest der Formel VI oder VI′
$R^{14}$ wie in Anspruch 1 definiert
$R^{15}$ unsubstituiertes Phenyl oder Benzoyl oder $(C_1-C_5)$-Alkyl
$R^{16}$ wie in Anspruch 1 definiert.

3. Verfahren zum Herstellen von Verbindungen der Formel I, II oder III nach Anspruch 1, dadurch gekennzeichnet, daß man Elaiophylin oder eine Verbindung der Formel XII, in der $R^1$ und $R^{1'}$ gleich oder verschieden sind und einen Rest der Formel IV und/oder V darstellen mit $R^2$, $R^3$, $R^4$, $R^5$ und $R^7$ wie in Anspruch 1 definiert,
umsetzt mit Verbindungen XIII, XIV oder XV

$R^{14}CHN_2$ (XIII)

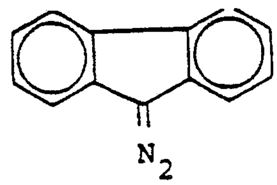

(XIV)

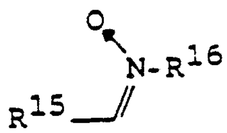

(XV)

worin $R^{14}$, $R^{15}$ und $R^{16}$ wie in Anspruch 1 definiert sind.

4. Verbindung I, II oder III nach Anspruch 1 zur Anwendung als Tierarzneimittel.

5. Verbindung I, II oder III nach Anspruch 1 zur Anwendung als Tierarzneimittel mit anthelminthischer Wirkung.

6. Verwendung einer Verbindung I, II oder III nach Anspruch 1 zur Herstellung eines Tierarzneimittels.

7. Verwendung einer Verbindung I, II oder III nach Anspruch 1 zur Herstellung eines Tierarzneimittels mit anthelminthischer Wirkung.

Europäisches Patentamt

# EUROPÄISCHER RECHERCHENBERICHT

Nummer der Anmeldung

EP 89106971.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 297 523 (HOECHST AG) * Zusammenfassung; Seite 5 * | 1,4-7 | C 07 H 17/04<br>C 07 H 15/26<br>A 61 K 31/71 |
| A | CHEMICAL ABSTRACTS, Band 106, Nr. 9, 2. März 1987, Columbus, Ohio, USA YOKURA SUSUMU et al. "Azalomy--cin B derivatives as anti--ulcer agents and antibiotics" Seite 646, Zusammenfassung-Nr. 67 626j & Jpn. Kokai Tokkyo Koho JP 61-36 295 | 1,4,6 | |
| A | B.W. BYCROFT et al. "Dictio--nary of Antibiotics and Re--lated Substances", 1988 CHAPMAN AND HALL LTD., London, New York Seite 300 * Seite 300: Elaiophylin * | 1,4,6 | RECHERCHIERTE SACHGEBIETE (Int. Cl.4)<br>C 07 H 17/00<br>C 07 H 15/00 |

Der vorliegende Recherchenbericht wurde für alle Patentansprüche erstellt.

| Recherchenort | Abschlußdatum der Recherche | Prüfer |
|---|---|---|
| WIEN | 11-07-1989 | JANISCH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument